# EUROPEAN PATENT APPLICATION

(11) **EP 0 620 014 A2**
(43) Date of publication of application: **19.10.1994**
(21) Application number: 94302497.6
(22) Date of filing: 08.04.1994
(51) Int. Cl.: A61L 9/01, C05F 3/00, A61L 11/00, A61L 9/04

(54) **Control of ammonia emission and odour**

(30) Priority: 14.04.1993 GB 9307651
(71) Applicant: VERDUGT B.V., NL-4000 AB Tiel (NL)
(72) Inventor: Van Ooijen, Johannes Adrianus Cornelis, Nl-4000 AB Tiel (NL)
(74) Representative: Krishnan, Suryanarayana Kalyana

(57) **Abstract**

This invention relates to a method of controlling or preventing ammonia emission from waste material capable of generating ammonia as such or after degradation over a period, said process comprising applying on the waste material an effective amount of a composition comprising a naturally occuring oil which is substantially immiscible with water.

## Description

This invention relates to a method of controlling and/or preventing emission of odoriferous ammonia from organic wastes, especially animal excrements and manure, using a novel composition.

Ammonia is the by-product from the degradation of urea by enzymes such as *urease,* which urea is in turn farmed by the degradation of uric acid. In poultry houses and in pig farms in general, the concentration of ammonia in the atmosphere can be sufficiently high for it not only to be a nuisance but also can be the cause of eg ammonia blindness in poultry. This in turn can put poultry or other animals off their feed and hence cause loss of production. In the case of pigs and piglets, presence of excess ammonia in the atmosphere where these are housed can cause respiratory illnesses. The same applies to other animals, be they cattle in a farm or domestic pets such as cats, dogs, birds, hamsters or guinea pigs. In all these cases there is a likelihood of excreta accumulating which is the prime cause of ammonia build up. The presence of excess ammonia in the atmosphere can also be harmful to farmworkers who have of necessity to handle such materials in order to produce good quality manure which in itself can give off ammonia due to degradation. In addition to the above, ammonia is one of the sources of acid rain.

Many methods have been tried to eliminate/control ammonia emission or to mask such odour but with limited success because the agents used for such purpose are either themselves odoriferous or are unpleasant to the handlers of such agents. Use of masking agents such as perfumes etc have also been unsuccessful and expensive.

For instance, US-A-3944908 describes the use of a biological fertilizer produced by treating sewage sedimentation sludge with sulphite waste liquor and the treated product is mixed with pulverised vegetable matter. Mineral acids such as sulphuric acid and hydrochloric acid have been used to control odours (cf. US-A-124041). US-A-3989498 describes the use of a mixture of glacial acetic acid and amyl alcohol for this purpose, the latter having a deodorising effect. US-A-5039481 describes a method of scavenging ammonia released into the air by decomposition of livestock excrement using a scavenging composition comprising a polycarboxylic acid such as oxalic acid; 1,2-, 1,3- or 1,4-cyclohexane dicarboxylic acid; or polyacrylic acid. These acids optionally contain solubilizing agents, eg water or a glycol, fragrances and an adsorbent which may be sawdust or rice hulls. The abstract of JP-A-85015331 is similar to the last document except that the composition comprises a mixture of oxalic, adipic or tartaric acid with a compost obtained from aerobic fermentation of sewages, faeces etc. US-A-4405354 also describes a method of supressing and limiting the ammoniacal odour of organic wastes by contacting the waste with an anti-ammoniacal agent which comprises a control component selected from (a) monobasic salts of dibasic acids, or, mono-, di- or tri- basic acids etc, the control component having a dissociation constant ( pKa) less than that of aqueous ammonia.

One of the attendant problems of the waste materials capable of generating ammonia is that these are usually exposed to the elements and for example, during rainfall, the agent used for absorbing ammonia is likely to be leached away thereby rendering the protection method substantially inadequate. This is particularly likely if the acidic agents used are eg sulphuric acid, hydrochloric acid, or fatty acids such as formic, acetic and propionic acid which are all completely miscible with water.

Thus, most of the expedients described above are either expensive or ineffective due to inadequate coverage of the required surface or due to their susceptibility to be leached by water or rain; in addition some of the acids such as formic, acetic or propionc acid are themselves significantly odorous and corrosive and are hence difficult to handle in the relevant environment.

It has now been found that the above problems can be mitigated by using naturally occuring oils which are substantially immiscible with water.

Accordingly, the present invention is a method of controlling or preventing ammonia emission from waste material capable of generating ammonia as such or after degradation over a period, said process comprising applying on the waste material an effective amount of a composition comprising a naturally occuring oil which is substantially immiscible with water.

The waste material referred to herein can be animal or human excreta or manure or compost produced therefrom.

The naturally occuring oils that may be used include those which can be sourced either from animals, whether land or marine, or from plants. Examples of oily materials that may be sourced from animals include lard oil, tallow from beef or mutton, Neat's foot oil, whale oil and sperm oil. Examples of oils that may be sourced from plant materials include corn oil, cotton seed oil, linseed oil, neem oil, niger-seed oil, olive oil, palm oil, peanut oil, poppy-seed oil, rapeseed oil, safflower oil, sesame oil, soybean oil, sunflower-seed oil and wheat-germ oil. Of these, rapeseed oil and soybean oil are preferred. These oils contain esters of saturated and/or unsaturated fatty acids which have 12 or more carbon atoms. These oils may be used as such or admixed with other components which are capable of counter-acting the effects of ammonia. For instance, these oils may be combined with aliphatic carboxylic acids which are substantially immiscible with water and are suitably the higher fatty acids which have at least 14 carbon atoms, preferably from 16-24 carbon atoms. Specific examples of such fatty acids include:
myristic acid - CH₃.(CH₂)₁₂.COOH
palmitic acid - CH₃.(CH₂)₁₄.COOH
stearic acid - CH₃.(CH₂)₁₆.COOH
arachidic acid - CH₃.(CH₂)₁₈.COOH
palmitoleic acid - CH₃.(CH₂)₅.CH=CH.(CH₂)₇.COOH;
oleic acid - CH₃.(CH₂)₇.CH=CH.(CH₂)₇.COOH;
ricinoleic acid - CH₃.(CH₂)₅CH(OH).CH₂.CH=CH.(CH₂)₇.COOH;
petroselinic acid - CH₃.(CH₂)₁₀.CH=CH.(CH₂)₄.COOH;
vaccenic acid - CH₃.(CH₂)₅.CH=CH.(CH₂)₉.COOH;
linoleic acid - CH₃.(CH₂)₄.CH=CH.CH₂.CH=CH.(CH₂)₇.COOH;
linolenic acid - CH₃.CH₂CH=CH.CH₂.CH=CH.CH₂.CH=CH(CH₂)₇.COOH;
eliostearic acid - CH₃.(CH₂)₃.(CH=CH)₃.(CH₂)₇.COOH;
licanic acid - CH₃.(CH₂)₃.(CH=CH)₃.(CH₂)₄.CO(CH₂)₂.COOH;
parinaric acid - CH₃.CH₂.(CH=CH)₄.(CH₂)₇.COOH;
tariric acid - CH₃.(CH₂)₇.C≡C.(CH₂)₇.COOH;
gadoleic acid - CH₃.(CH₂)₉.CH=CH.(CH₂)₇.COOH;
arachidonic acid - CH₃.(CH₂)₄.(CH=CH.CH₂)₄.(CH₂)₂.COOH;
cetoleic acid - CH₃.(CH₂)₉.CH=CH.(CH₂)₉.COOH;
erucic acid - CH₃.(CH₂)₇.CH=CH(CH₂)₁₁.COOH; and
nervonic acid - CH₃.(CH₂)₇.CH=CH(CH₂)₁₃.COOH.

Of the substantially water-immiscible carboxylic acids, it is preferable to use the unsaturated aliphatic fatty acids as an adjuvant for the naturally occuring oils.

It is preferable to use oils or oil formulation which are liquids at ambient temperatures since this would facilitate spreading of the oil (formulation) over the surface of the waste material to be treated more evenly. It has been found that even in the case of the oils which are not liquids at ambient temperatures, the melting point may be reduced by mixing them with other oils or carboxylic acids which have a very low melting point. In fact, the use of these oils, which are substantially immiscible with water, prevents the oils being leached out from the surface of waste materials treated therewith when exposed to rain or other sources of water. This ensures and extends the continuous control and prevention of ammonia emission from the treated waste material.

The naturally occuring oils such as rapeseed and soybean oils are substantially non-toxic and not only very safe to handle by the operatives who apply the oils on the waste material but is also safe with respect to any animals which may accidentally or otherwise come in contact therewith.

If desired, the oils or oil formulations may be applied on the surface of the waste material either as such or, in particulate, pelletised or granular form by impregnating an adsorbent with the oil or oil formulation. The use of natural oils such as rapeseed oil, soybean oil or sunflower-seed oil has the added advantage that these oils provide a secondary physical barrier against the escape of ammonia by forming a film on the surface of the waste material treated. Where the oils or oil formulations are used in a particulate, pelletised or granular form, the adsorbent materials that may be used for this purpose include saw dust, rice husks, bark, straw, ground peanut shells, alfalfa, diatomaceous earth, calcium sulphate, perlite, vermiculite and other siliceous materials.

The mode of application of the oils on the waste material would depend upon the type of waste material and the point of use. For instance, in the case of domestic pets eg cats, dogs, birds etc, it would be preferable to use these in adsorbed particulate, pelletised or granular form in order to facilitate handling of the material. However, in the case of a farm where vast quantities of such waste materials are produced by eg cattle, pigs and poultry, and the waste is intended to be or used in the production of compost or manure, the liquid form of the oil (formulation) is preferred to maximise the area covered.

Thus according to a further embodiment, the present invention is an ammonia emission control composition consisting essentially of an effective amount of a naturally occuring oil which is substantially immiscible with water.

Typically such oil (formulation) is preferably applied to the waste material in an amount which substantially completely covers the exposed surface of the waste material from which ammonia emission occurs or is likely to occur.

In order to make the oils and oil formulations of the present invention even more user friendly, such compositions may be admixed with fragrances or deodorants such as eg camphor, menthol or other phenolic materials, alcohols such as amyl alcohol or esters such as eg amyl acetate prior to application on the waste material.

The present invention is further illustrated with reference to the following Example:

### EXAMPLE 1:

10 litres of a 0.15% ammonia solution in water was placed in a can in a working fume cupboard. The total exposed surface area of the solution was 804 cm². With Draeger tubes the ammonia concentration above the solution was measured before and after the surface of the solution was covered with 100g of soya bean [?] oil.

Before covering with the soya bean [?] oil, the ammonia concentration in the air above the exposed surface of the solution was 65-70ppm (measured in triplicate). After covering with the soya bean [?] oil, the ammonia concentration was measured again and found to be only 10 ppm.

The treated sample of the ammonia solution was stored in a closed jar at room temperature for a week and the ammonia emission measured again. No increase in ammonia emission was found. The sample jar was shaken and the ammonia concentration in the air above the exposed surface of the solution was measured again. There was no increase in the ammonia concentration after shaking.

The above result shows that covering the surface of an ammonia emitting substance with a naturally occuring oil according to the present invention drastically reduces ammonia emission from said substance.

## Claims

1. A method of controlling or preventing ammonia emission from waste material capable of generating ammonia as such or after degradation over a period, said process comprising applying on the waste material an effective amount of a composition comprising a naturally occuring oil which is substantially immiscible with water.

2. A method according to claim 1 wherein the waste material is an animal or human excreta or manure or compost produced therefrom.

3. A method according to claim 1 or 2 wherein the naturally occuring oils used is sourced either from animals, whether land or marine, or from plants.

4. A method according to any one of the preceding claims wherein the naturally occuring oil is that sourced from animals and is selected from lard oil, tallow from beef or mutton, Neat's foot oil, whale oil and sperm oil.

5. A method according to any one of the preceding claims 1-3 wherein the naturally occuring oil is that sourced from plant materials and is selected from corn oil, cotton seed oil, linseed oil, neem oil, niger-seed oil, olive oil, palm oil, peanut oil, poppy-seed oil, rapeseed oil, safflower oil, sesame oil, soybean oil, sunflower-seed oil and wheat-germ oil.

6. A method according to any one of the preceding claims wherein the naturally occuring oils are admixed with aliphatic carboxylic acids which are substantially immiscible with water prior to application on the waste materials.

7. A method according to claim 6 wherein the aliphatic carboxylic acids are the higher fatty acids which have at least 14 carbon atoms.

8. A method according to claim 6 or 7 wherein the aliphatic carboxylic acids are selected from the group consisting of:
myristic acid - CH₃.(CH₂)₁₂.COOH
palmitic acid - CH₃.(CH₂)₁₄.COOH
stearic acid - CH₃.(CH₂)₁₆.COOH
arachidic acid - CH₃.(CH₂)₁₈.COOH
palmitoleic acid - CH₃.(CH₂)₅.CH=CH.(CH₂)₇.COOH;
oleic acid - CH₃.(CH₂)₇.CH=CH.(CH₂)₇.COOH;
ricinoleic acid - CH₃.(CH₂)₅CH(OH).CH₂.CH=CH.(CH₂)₇.COOH;
petroselinic acid - CH₃.(CH₂)₁₀.CH=CH.(CH₂)₄.COOH;
vaccenic acid - CH₃.(CH₂)₅.CH=CH.(CH₂)₉.COOH;
linoleic acid - CH₃.(CH₂)₄.CH=CH.CH₂.CH=CH.(CH₂)₇.COOH;
linolenic acid - CH₃.CH₂CH=CH.CH₂.CH=CH.CH₂.CH=CH(CH₂)₇.COOH;
eliostearic acid - CH₃.(CH₂)₃.(CH=CH)₃.(CH₂)₇.COOH;
licanic acid - CH₃.(CH₂)₃.(CH=CH)₃.(CH₂)₄.CO(CH₂)₂.COOH;
parinaric acid - CH₃.CH₂.(CH=CH)₄.(CH₂)₇.COOH;
tariric acid - CH₃.(CH₂)₇.C≡C.(CH₂)₇.COOH;
gadoleic acid - CH₃.(CH₂)₉.CH=CH.(CH₂)₇.COOH;
arachidonic acid - CH₃.(CH₂)₄.(CH=CH.CH₂)₄.(CH₂)₂.COOH;
cetoleic acid - CH₃.(CH₂)₉.CH=CH.(CH₂)₉.COOH;
erucic acid - CH₃.(CH₂)₇.CH=CH(CH₂)₁₁.COOH; and
nervonic acid - CH₃.(CH₂)₇.CH=CH(CH₂)₁₃.COOH.

9. A method according to any one of the preceding claims wherein the naturally occuring oils are liquids at ambient temperatures.

10. A method according to any one of the preceding claims wherein the naturally occuring oils or an admixture thereof with aliphatic carboxylic acids is applied on the surface of the waste material either as such or, in particulate, pelletised or granular form by impregnating an adsorbent with the oil or the admixture.

11. A method according to claim 10 wherein the naturally occuring oils or admixtures thereof with aliphatic caboxylic acids are impregnated on an adsorbent material that is selected from the group consisting of saw dust, rice husks, bark, straw, ground peanut shells, alfalfa, diatomaceous earth, calcium sulphate, perlite, vermiculite and other siliceous materials.
